# EUROPEAN PATENT SPECIFICATION

(11) **EP 2 102 329 B1**
(45) Date of publication and mention of the grant of the patent: **26.05.2010**
(21) Application number: 07859506.3
(22) Date of filing: 21.12.2007
(51) Int. Cl.: C12N 5/071, C12N 5/09

(54) **DIFFERENTIATED IMMORTALISED CELL LINES CAPABLE OF PRODUCING ALBUMIN AND BLOOD COAGULATION FACTORS, METHODS OF PREPARING THEREOF FROM A LEUKAEMIA CELL LINE AND USES THEREOF**
DIFFERENZIERTE IMMORTALISIERTE ZELLLINIEN MIT FÄHIGKEIT ZUR PRODUKTION VON ALBUMIN UND BLUTGERINNUNGSFAKTOREN, VERFAHREN ZUR HERSTELLUNG DAVON AUS EINER LEUKÄMIEZELLLINIE SOWIE VERWENDUNGEN DAVON
LIGNÉES CELLULAIRES IMMORTALISÉES DIFFÉRENCIÉES CAPABLES DE PRODUIRE DE L'ALBUMINE ET DES FACTEURS DE COAGULATION SANGUINE, PROCÉDÉS DE PRÉPARATION DE CELLES-CI À PARTIR D'UNE LIGNÉE CELLULAIRE LEUCÉMIQUE, ET UTILISATIONS DE CELLE-CI

(30) Priority: 29.12.2006 IT TO20060937
(43) Date of publication of application: 23.09.2009
(73) Proprietor: Medestea Biotech S.P.A., 10121 Torino (IT)
(72) Inventor: GENNERO, Luisa, I-10127 Torino (IT); PONZETTO, Antonio, I-10024 Moncalieri (Torino) (IT); PESCARMONA, Giampiero, I-10123 Torino (IT); SAVARINO, Andrea, I-10048 Vinovo (Torino) (IT); MERIZZI, Gianfranco, I-10128 Torino (IT)
(74) Representative: Comoglio, Elena
(86) International application number: PCT/IB2007/055287
(87) International publication number: WO 2008/081388

(56) References cited:
- WO-A-2007/057853
- US-A- 5 290 684
- US-A1- 2005 221 483
- TSUCHYA S ET AL: "ESTABLISHMENT AND CHARACTERIZATION OF A HUMAN ACUTE MONOCYTIC LEUKEMIA CELL LINE THP-1" INTERNATIONAL JOURNAL OF CANCER, NEW YORK, NY, US, vol. 26, no. 2, 1 January 1980 (1980-01-01), pages 171-176, XP002429869 ISSN: 0020-7136

## Description

The present invention relates to differentiated cell lines capable of producing albumin and blood coagulation factors derived from a leukaemia cell line, preferably from the commercially available THP 1 cell line.

The present invention makes use of pluripotent stem cells (hereinafter referred to as "PSCs-THP1", *i.e*. Pluripotent Stem Cells-THP1) derived from the THP1 leukaemic cell line by subcloning. In particular, PSCs-THP1 cells are obtained from the THP1 cell line which is selected by the sorter technique uniquely for THP1-CD34+/CD14+/CD90+ cells.

PSCs-THP1, that is to say THP1-CD34+/CD14+/CD90+ pluripotent stem cells, are not selected with the aid of external induction, such as culture media or growth factors, but rather solely by means of the isolation of components showing a marked positivity for at least three stem cell surface clusters, namely the markers CD34, CD14 and CD90.

PSCs-THP1-CD34+/CD14+/CD90+ cells are then induced to differentiate into various phenotypes characteristic of human tissues, particularly the human hepatocytic phenotype.

Thus, the present invention also relates to methods for obtaining a pluripotent stem cell line starting from a leukaemia cell line (preferably THP1), methods of differentiating said pluripotent stem cell line and the related differentiated cell lines characterised by specific phenotypes, particularly the human hepatocytic phenotype, as well as the use of the cell line with a human hepatocytic phenotype derived from the pluripotent stem cell line PSC-THP1-CD34+/CD14+/CD90+ for the production of albumin and blood coagulation factors, and the associated therapeutic applications.

### TECHNICAL BACKGROUND OF THE INVENTION

Stem cells are fundamentally important for all living organisms and are characterised by two peculiarities which distinguish them from other cell types. Firstly, they constitute a continuous and inexhaustible source of non-specialised cells capable of replicating. Secondly, stem cells have the ability to become specialised and can differentiate into different cell types in response to localised signals. Said event occurs in circumstances where a damaged body tissue requires it. Under certain physiological or experimental conditions, stem cells are induced to undergo changes and to assume specialised functions (e.g. myocytes in muscle, or hepatocytes in liver etc.). Thus, stem cells have the ability to multiply and also to undergo changes so as to generate many different cell types, and ultimately, have the potential ability to replace damaged tissues [1-15].

One commonly accepted definition of "stem cell" is that of a cell with two characteristics [2]:
1) the ability for unlimited or prolonged self-renewal, *i.e*. the ability for long-term reproduction without differentiation;
2) the ability to give rise to transient progenitor cells, with limited proliferative ability, from which descend populations of highly differentiated cells (hepatic, nervous, muscle, blood, intestinal etc.).

The ability of stem cells to differentiate into specific tissues varies depending on the origin of the cells and the developmental stage of the organism from which they are extracted. Thus, there are several types of stem cells [2]:

Pluripotent cells, in the sense that they possess the potential to differentiate into any adult animal cell type, but not totipotent cells capable of giving rise to an embryo. By the fourth or fifth day after fertilisation (morula stage) the embryo still consists of identical embryonic cells, but said cells can no longer form an embryo. From the fifth and sixth day post-fertilisation (blastocyst stage, with the presence of a hundred or more cells) a spherical cavity forms inside the morula, from the external cellular mass of which begin to differentiate the cells that will form the placenta and the membranes surrounding the embryo, while from the internal mass begin to differentiate the cells that will form the true embryo. These latter cells, isolated from the cell mass from the internal cavity, are precisely the pluripotent stem cells. Indeed, if such cells are transferred into a uterus, they would not have the ability to implant and develop, because embryo development cannot progress unless synchronised with those of the placenta, from which they derive nutrition. Pluripotent stem cells have also been identified in various tissues in adult humans (bone marrow, liver, synovia, dental pulp, heart, intestine, peripheral blood, etc.).

However, embryonic stem cells have great ability for proliferation and differentiation into an enormous number of different cell types, including bipotent hepatic oval cells and hepatocytes. They are the most promising for the large scale production of hepatocytes. But, just as with foetal cells, their usefulness is limited by ethical considerations, difficulty of isolation, the possibility of rejection and the risk of inducing tumour development.

From research into adult stem cells over the last thirty years, it has emerged clearly that stem cells are present in many adult tissues, but that they are only capable of giving rise to cells of a given tissue type. *I.e*. the possibility of their reprogramming has not been considered. However, more recently, pluripotent stem cells have also been discovered in various human tissues - in bone marrow, the brain, in the mesenchyme of various organs, in umbilical cord blood and in peripheral blood - capable of giving rise to several cell types, predominantly blood, muscle and nerve. How to recognise them, select them, how to sustain their development and how to induce them to form various mature cell types by means of growth factors and other regulatory proteins, has been observed. It is sufficient to mention that, in humans, the bone marrow stem cells, from which other blood cell lines are formed, have the molecules CD14+ and/or CD29+ and/or CD34+ and/or CD44+ and/or CD45+ and/or CD71+ and/or CD90+ and/or CD105+ and/or CD117+ as recognition markers, and when purified, they are capable of reconstituting the entire population of blood cells in patients who have received ablative doses of radiation and chemotherapy, and they can do this at a speed proportional to the quantity of cells used [1].

It is known that for transplanting stem cells (CD14+ and/or CD29+ and/or CD34+ and/or CD44+ and/or CD45+and/or CD71+ and/or CD90+ and/or CD105+ and/or CD117+ haematopoietic progenitors) derived from umbilical cord or other sources, donor-recipient compatibility is essential. For this reason, a number of bone marrow and placental blood banks have been established. In the case of an allogenic transplant, the search for a compatible donor may take a rather long time; on the other hand, in the case of an autologous transplant, i.e. stem cells from the same transplant patient (from marrow or peripheral blood), the stem cells must fist be removed, isolated, cultured, expanded and stored intact, even for quite some time.

Indeed, progenitor stem cell (CD14+ and/or CD29+ and/or CD34+ and/or CD44+ and/or CD45+ and/or CD71+ and/or CD90+ and/or CD105+ and/or CD117+) viability has important effects on the ability of the transplant to take root [1-2].

In adult humans,various tissues including bone marrow, peripheral blood; liver, intestine, dental pulp, synovia, etc., contain stem cells capable of differentiating into adult hepatocytes. The therapeutic use of such cells is very interesting, given the ease with which they can be cultured (*ex vivo*). Since they are derived from the patient, they are always compatible, and have no associated ethical problems [1].

Adult bone marrow contains haematopoietic (from blood) and stromal (which form the scaffolding of organs) progenitor cells. Among the haematopoietic cell lines, over the long term, the so-called "HSC" cells give rise to various cell lines, including endothelial cell lines. Mesenchymal stromal cells have the same capability [2].

Adult blood contains haematopoietic progenitor stem cells. According to the work by Huberman, 2003, particular "HSC" (CD14+ and/or CD34+) cells can be identified among haematopoietic cells that are pluripotent, which when suitably stimulated using growth factors, can differentiate and specialise into cells typical of a number of body parts, including the liver [1-2].

In 2003, Huberman *et al.* [1] identified a group of cells (CD14+ and/or CD34+) present in the human peripheral blood monocytic fraction, with stem cell-like and pluripotent characteristics (PSCs, Pluripotent Stem Cells), *i.e*.: it was shown how cells can be induced to acquire macrophagic, lymphocytic, epithelial, endothelial, neuronal and hepatocytic phenotypes without the need to fuse with pre-existing mature tissue.

Unfortunately, these cells, grown in culture medium supplemented with growth factors, are not immortal *in vitro,* and within 30-40 days post isolation they become senescent and die. Where these cells are grown in medium free from growth factors, this phenomenon is accelerated *i.e.,* within 7-8 days post-isolation *in vitro,* they become senescent and die.

Hence, the pluripotent circulating CD14+/CD34+ monocytic stem cells discovered by Huberman *et al*. [1]*,* and described in patent WO2006044842, may be defined as "MORTAL" [1]. Furthermore, studies on cell death, conducted by ourselves, according to the method of Huberman *et al.,* on the population of PSCs isolated from the peripheral blood of 10 volunteers, have revealed the increased importance of the protein p27 (a nuclear protein which controls apoptosis) at the thirtieth day in cells treated with growth factors, and at the seventh day in cells treated with growth factor free media; this data would appear to confirm the existence of "programmed cell death" that depends on progressive cellular senescence.

These observations relating to cell death, senescence and apoptosis are also advanced in European patent applications EP1436381B1 or EP1506999 [Kremer Bernd Karl Friedrich (DE); Faendrich Fred (DE); Ruhnke Maren) (DE)] from Blasticon Biotechnologische Furschung GmbH (US2005/0233447) entitled "Dedifferentiated, programmable stem cells of monocytic origin, and their production and use"; according to *Kremer et al.,* dedifferentiated stem cells of human monocytic origin are characterised by the presence of the membrane-associated monocyte-specific surface antigen CD 14, and by the presence of at least one pluripotent marker selected from CD117, CD123 and CD135.

The afore-cited patents refer to obtaining monocytes by means of separating human blood, and, in paragraph 59, by direct isolation from internal organs if separation from human blood is not possible, such as in the case of anaemia or leukaemia patients. Therefore, on the basis of the content of this patent, it is possible to assume that the cells in question are prone, since they are non-tumour cells, to progressive cellular senescence dependent "programmed cell death".

### DESCRIPTION OF THE INVENTION

The object of the present invention is that of providing a pluripotent stem cell line which is simple to acquire, immortal (immortal because it is leukaemia-derived), stable over time and easy to differentiate in order to obtain specialised cell lines suitable for use for the production of biological molecules.

According to the present invention, said object is achieved thanks to the solution specifically claimed in the following claims. The claims constitute an integral part of the technical teaching provided herein in relation to the invention.

One particularly preferred embodiment of the present invention relates to an immortal leukaemia pluripotent stem cell line derived from an immortal human monocytic leukaemia cell line, preferably the human cell line THP1.

In a further embodiment, the invention concerns a method of conditioning and stimulating a human monocytoid leukaemia cell line to become an immortal leukaemia pluripotent stem cell line. More specifically, and in a particular embodiment, the immortal monocytoid leukaemia cell line is the THP1 cell line and it is selected by mono-di-triclonal amplification and expansion of naturally immature immortal THP1 leukaemia stem calls displaying at least three cell surface markers which indicate potential pluripotent stem cell nature, namely CD34+, CD14+ and CD90+; thereby obtaining the PSCs-THP1-CD34+eCD14+eCD90+ cell line.

The immortal PSCs-THR1-CD34+eCD14+eCD90+ cell line concerns immortal cell lines derived from a pluripotent immortal stem cell line having the phenotype of cell strains which are characteristic of human tissues, particularly a human hepatocytic phenotype.

In another preferred embodiment, the present invention relates to a method of inducing the differentiation of an immortal pluripotent stem cell line into derivative (differentiated) immortal cell lines having the phenotypes of cell strains from human tissues of interest. In particular, the present invention relates to a method of differentiating the immortal cell line PSCs-THP1-CD34+eCD14+eCD90+ into a derivative immortal cell line having two main features: a mature hepatocytic monoclonal phenotype and immortality.

The PSCs-TBP1-CD34+eCD14+eCD90+ cells induced to differentiate into hepatocytes become mature hepatocytes (PSC-THP1-EP), and as such they can be used in diagnostics, research and the prevention and treatment of chronic and acute liver disorders.

In a particularly preferred embodiment, the immortal mature human hepatocytic cell line according to the invention is the cell line designated as PSC-THP1-EP, deposited with the Advanced Biotechnology Centre - Interlab Cell Line Collection (ICLC), Genoa, Italy, under accession number ICLC PD No. 06005 on 10 November 2006.

The immortal PSC-THP1-EP cell line has a mature hepatocytic phenotype. The characterisation of certain cell markers of this cell line are listed by way of example: human HLA-DR1 MHC class II, CD34+, CD44+, CD68+, CD71+, CD81+, CD90+, CD105+, CD117+, TLR5, CDw328, CDw156c; CD172g , LDL Rec (LDL5/6+)+, GATA-4+, HNF-1alfa+, HNF-3beta+, IL-1 RT 1+, CK8+, CK18+, CK7-/+, CK19--/+, c-Met, Alphafetoprotein--/+, ALBUMIN+.

Said immortal cell line, having a mature hepatocytic PSC-THP1-EP phenotype, shows the following metabolic features: cell cycle with doubling every two - three days, secretion of albumin and blood coagulation factors from the third up to the fifth day of culture.

Preferably, from the 72th to 120th hour in culture upon replacement of the culture medium, said PSC-THP1-EP cell line shows a characteristic secretion of albumin and blood coagulation factors (albumin: 30-60 g/l every 72-120 hours of culture upon replacement of the culture medium; blood coagulation factors between 5% and 90% of the content present in circulating human blood every 72-120 hours of culture upon replacement of the culture medium).

Furthermore, by means of a number of sequential splits of the PSC-THP1-EP cell line (ICLC PD No. 06005, 10 November 2006), a new cell population has been selected, i.e. the immortal cell line designated as PSC-THP1-EP-FAST having a mature hepatocytic phenotype, isolated from a colony with particular metabolic features: rapid cell cycle with doubling every six to eight hours, secretion of albumin and blood coagulation factors from the third hour upon replacement of the culture medium, with a production peak between hours eight and twelve (albumin: 8-10 g/l every 8-12 hours of culture upon replacement of the culture medium; blood coagulation factors between 5% and 90% of the content present in circulating human blood every 8-12 hours of culture upon replacement of the culture medium).

The immortal human mature hepatocyte cell line according to the invention designated as PSC-THP1-EP-FAST was deposited with the Advanced Biotechnology Centre - Interlab Cell Line Collection, Genoa, Italy on 15 December 2006, under accession number ICLC PD No. 06008.

The immortal cell line designated as PSC-THP1-EP-FAST has properties which are typical of pluripotent stem cells differentiated into mature hepatocytes. Moreover, it retains the original immortalisation conferred by being derived from a leukaemia cell line, and it is selected on the basis of some particular metabolic features: rapid cell cycle; doubling every six to eight hours with replacement of the culture medium every twelve hours (replacement of 50-70% of the culture medium every twelve hours); rapid metabolism and catabolism (8-12 hours).

The characterisation of certain cell markers of this cell line is listed by way of example: human HLA-DR1 MHC class II, CD34+,CD44+, CD68+, CD71+, CD81+, CD90+, CD105+, CD117+, TLR5, CDw328, CDw156c, CD172g , LDL Rec (LDL5/6+)+, GATA-4+, HNF-1alfa+, HNF-3beta+, IL-1 RT 1+, CK8+, CK18+, CK7-/+, CK19--/+, c-Met, Alphafetoprotein--/+, ALBUMIN+.

As mentioned above, the immortal cell lines having the above-described mature hepatocytic phenotype are derived from the PSCs-THP1-CD34+eCD14+eCD90+ immortal pluripotent stem cell line for the production of albumin and blood coagulation factors.

Furthermore, by polyethylene glycol (PEG)-mediated cell fusion between the human HepG2 cell line (available from the American Type Culture Collection) and the PSC-THP1-EP cell line, a new cell line (PSC-THP1-HEP) has been obtained, having properties which are typical of pluripotent stem cells differentiated into hepatocytes and into hepatoblasts, preserving the original immortalisation conferred by being derived from the leukaemia cell line.

In a particularly preferred embodiment, the immortal human hepatocytic cell line according to the invention is the cell line designated as PSC-THP1-HEP, deposited with the Advanced Biotechnology Centre - Interlab Cell Line Collection, Genoa, Italy, on 15 December 2006, under accession number ICLC PD No. 06006.

In particular, the present invention concerns the use of said PSC-THP1-HEP immortal cell line with properties typical of pluripotent stem cells differentiated into mature and immature hepatocytes preserving the original immortalisation conferred by being derived from leukaemia cell lines.

The karyotype of the PSC-THP1-HEP immortal cell line of the invention, with mature and immature hepatocytic phenotype, is illustrated below by way of example: "50∼52,XY/XXY,-Y, der(1)?t(1;12)(p32;q13),+der(1)del(1)(p22p3?4)add(1)(q21),+del(6)(p21?1),+del(6)(p21?1 ),12,14,+der(14)?t(3;14)(p11;p12),+del(17)(p11),18,+mar1,+mar2,+mar3,+mar4".

Said immortal cell line, having a mature hepatocytic PSC-THP1-HEP phenotype, has the following metabolic features: cell cycle with doubling every two - three days, secretion of albumin and blood coagulation factors from the third up to the seventh day of culture.

Preferably, said PSC-THP1-HEP cell line shows, between the 72th and the 144th hour in culture, upon replacement of the culture medium, a characteristic secretion of albumin and blood coagulation factors (albumin: 30-60 g/l every 72-144 hours of culture upon replacement of the culture medium; blood coagulation factors between 5% and 90% of the content present in circulating human blood every 72-144 hours of culture, upon replacement of the culture medium).

Furthermore, by a preferred PEG-mediated cell fusion between the human HepG2 cell line and the PSC-THP1-EP cell line and primary mature human hepatocytes, a new cell line has been obtained having properties which are typical of pluripotent stem cells differentiated into mature hepatocytes and preserving the original immortalisation conferred by being derived from leukaemia cell lines.

In a particularly preferred embodiment, the mature immortal human hepatocytic cell line according to the invention is the cell line designated as PSC-THP1-EPEP, deposited with the Advanced Biotechnology Centre - Interlab Cell Line Collection, Genoa, Italy, on 15 December 2006, under accession number ICLC PD No: 06007.

Said PSC-THP1-EPEP immortal cell line has properties typical of pluripotent stem cells differentiated into mature hepatocytes preserving the original immortalisation conferred by being derived from leukaemia cell lines.

The PSC-THP1-EPEP immortal cell line has a mature hepatocytic phenotype, the karyotype of which is listed by way of example:"50∼52,XY/XXY,Y,der(1)?t(1;12)(p32;q13),+der(1)del(1)(p22p3?4)add(1)(q21), +del(6)(p21?1),+del(6)(p21?1);12,14,+der(14)?t(3;14)(pl 1;p12),+del(17)(p11,+mar1;+mar 2,+mar3".

Said immortal cell line, with mature hepatocytic PSC-THP1-EPEP phenotype, has the following metabolic characteristics: cell cycle with doubling every two - three days, secretion of albumin and blood coagulation factors from the third up to the fifth day of culture.

Preferably, between the 72th and the 120th hour of culture upon replacement of the culture medium, the PSC-THP1-EPEP cell line of the invention shows a characteristic secretion of albumin and blood coagulation factors (albumin,: 30-60 g/l every 72-120 hours of culture upon replacement of the culture medium; blood coagulation factors between 5% and 90% of the content present in circulating human blood every 72-120 hours of culture, upon replacement of the culture medium).

Furthermore, the present invention concerns the use of albumin as a therapeutic agent and as a carrier for drugs or vaccines; said albumin is produced by the immortal cell line having a mature hepatocytic phenotype derived from the selection of clones of immortal pluripotent stem cells by sorting, in particular by isolating and expanding said PSCs-THP1-CD34+eCD14+eCD90+ cell line.

The immortal THP-1 cell line is a human monocytoid cell line (hence leukaemic and immortal), derived from the culture of cells (leukaemic monocytes) found in the peripheral blood of a one year old Japanese child affected with monocytic leukaemia (Tsuchiya *et al*.) [16]. Circulating monocytes from this child donor placed in culture did not expire and replicated with high frequency (every two days) *in vitro;* said monocytes had been rendered immortal due to their leukaemic origin.

Monocytic leukaemic cells are defined as "monocytoids", *i.e*. having phenotype features which are similar to those of the cells from which they are derived, the monocytes, but which are however not identical; indeed, monocytes are cells with a precise cellular morphology which undergo cell ageing (biological senescence) and death independently of external factors, whilst monocytoid leukaemic cells are immortalised by tumour genes, hence they never get old and have extremely variable morphology, strictly related to the environmental conditions.

An immortal cell population capable of perpetual and infinite replication, i.e. the THP-1 cell line, has been isolated from the monocytoids of the Japanese child [16]. Therefore, the appropriate definition for the immortal THP-1 cells is not that of monocytes, but, since they are leukaemic in nature, that of monocytoid cells, having the typical features of being highly immature and immortal [1-27].

By the expression immortal cell line is meant a group of cells which are capable, once placed in culture, of reproducing forever, without becoming senescent (biological ageing).

The PSCs-THP1-CD34+eCD14+eCD90+ immortal pluripotent stem cell line, derived from the THP1 immortal cell line, has been obtained by selecting from said THP1 immortal cell line, by *in vivo* sorting, those cells showing at least triple positivity for the stem cell surface markers designated as CD34+eCD14+eCD90+, thereby obtaining and expanding cells having features which are typical of pluripotent stem cells, but at the same time also preserving the extremely important feature of the immortality.

Subsequent conditioning of the immortal cell line PSCs-THP1-CD34+eCD14+eCD90+ allowed to obtain a cell line which is also immortal and has the typical functions of mature hepatocytes (PSC-THP1-EP). In particular, these immortal PSC-THP1-EP cells were induced to differentiate and thus acquire the morphology and functional features of mature hepatocytes in a homogeneous population free of immature elements.

Furthermore, by a number of sequential splits of the PSC-TBP1-EP cell line, a novel population was selected, i.e. the PSC-THP1-EP-FAST immortal cell line having a mature hepatocytic phenotype, isolated from a colony showing particular metabolic features.

Furthermore, by the preferred PEG-mediated cell fusion technique between the human HepG2 cell line and the PSC-THP1-EP cell line, a new cell line was obtained having properties which are typical of the pluripotent stem cells differentiated into hepatocytes and hepatoblasts and preserving the original immortalisation conferred by being derived from leukaemia cell lines (PSC-THP1-HEP).

Furthermore, by the preferred PEG-mediated cell fusion technique among the human HepG2 cell line and the PSC-THP1-EP cell line and primary mature human hepatocytes, a new cell line was obtained having properties which are typical of the pluripotent stem cells differentiated into mature hepatocytes and preserving the original immortalisation conferred by being derived from leukaemia cell lines (PSC-THP1-EPEP).

Hepatocytes are the functional liver cells which produce the plasma proteins responsible for maintaining homeostasis and coagulation.

The possibility of:
- inducing the differentiation of immortal PSCs-THP1-CD34+eCD14+eCD90+ cells into immortal mature hepatocytic cells (PSC-THP1-EP),
- selecting cellular subclones with precise and particular metabolic characteristics (PSC-THP1-EP-FAST),
- preparing novel stable mature hepatocytic cell lines by cell fusion (PSC-THP1-HEP and PSC-THP1-EPEP),
hence provides a continuous, stable and immortal cell system for the production of albumin and blood coagulation factors having the physico-chemical, biological and physiological characteristics of human albumin and human blood coagulation factors, thus becoming the source of choice for the production of such proteins, essential for the treatment of a number of disorders.

By way of example, with reference to albumin, it is possible to cite the following pathologies: liver failure, hypoalbuminaemia, hypovolemia, nephrosic syndrome, Menetrier's disease, entero-enteric fistulas, burns, hepatitis, hepatic fibrosis, cirrhosis, serious hydrosaline retention in liver cirrhosis or following paracentesis, in malabsorption syndromes.

By way of example, with reference to blood coagulation factors, it is possible to cite the following pathologies:
Haemorrhaging
congenital or acquired defects of the vascular wall;
thrombocytopenia or thrombocytopathy, *i.e*. platelet related disorders where the numbers can even be normal;
congenital or acquired deficiencies involving one or more blood coagulation factor, *i.e*.: Factor VIII or IX deficiency (haemophilia), combined Factor V and VIII deficiency, Factor XIII deficiency, antiplasmin, increased plasminogen activity and PAI (Plasminogen Activator Inhibitor) deficiency, isolated platelet factor 3 deficiency, dysfibrinogenemia, Von Willebrand's disease, potential factor XII deficiency, prekallikrein and high molecular weight kininogen deficiency;
hepatopathies, circulating anticoagulants, oral anticoagulant therapy, heparin, disseminated intravascular coagulation;
Excessive fibrinolysis mechanism activity Thrombosis
defects, generally acquired, of the vessel wall;
congenital or acquired natural coagulation inhibitor deficiencies (*e.g*. AT III, Protein C or Protein S deficiency;
significantly and persistently raised platelets;
fibrinolytic mechanism deficiency.

### DETAILED DESCRIPTION OF THE INVENTION

Purely by way of a non-limiting example, the present invention will be now described in detail with reference to several preferred embodiments.

PSCs-THP1-CD34+eCD14+eCD90+ cells have been committed towards specialization (in the sense of maturation and differentiation) and all of them homogeneously become cells having functions which are typical of mature hepatocytes (PSC-THP1-EP).

By way of example, the immortal PSC-THP1-EP cell line having a hepatocytic phenotype shows the following characterisation in relation to certain cell markers: human HLA-DR1 MHC class II, CD34+, CD44+, CD68+, CD71+, CD81+, CD90+, CD105+, CD117+, TLR5, CDw328, CDw156c, CD172g , LDL Rec (LDL5/6+)+, GATA-4+, HNF-1 alfa+, HNF-3beta+, IL-1 RT 1+, CK8+, CK18+, CK7-/+, CK19--/+, c-Met, Alphafetoprotein--/+, ALBUMIN+.

Said immortal cell line, having mature hepatocytic PSC-THP1-EP phenotype, shows the following metabolic features: cell cycle with doubling every two - three days, secretion of albumin and blood coagulation factors from the third up to the fifth day of culture.

Preferably, between the 72th and 120th hour of culture upon replacement of the culture medium, said PSC-THP1-EP cell line shows a characteristic secretion of albumin and blood coagulation factors (albumin: 30-60 g/l every 72-120 hours of culture upon replacement of the culture medium; blood coagulation factors between 5% and 90% of the content present in circulating human blood every 72-120 hours of culture, upon replacement of the culture medium).

Furthermore, by a number of sequential splits of the PSC-THP1-EP cell line (PD 06005 of 10 November 2006), a novel population was selected, the PSC-THP1-EP-FAST cell line, isolated from a colony having particular metabolic features such as: rapid cell cycle with doubling every six to eight hours, secretion of albumin and blood coagulation factors from the third hour following replacement of the culture medium, with a production peak between hours eight and twelve (albumin: 8-10g/l every 8-12 hours of culture upon replacement of the culture medium; blood coagulation factors between 5% and 90% of the content present in circulating human blood every 8-12 hours of culture, upon replacement of the culture medium).

In particular, said PSC-THP1-EP-FAST immortal cell line having properties typical of pluripotent stem cells differentiated into mature hepatocytes and retaining the original immortalisation conferred by being derived from leukaemia cell lines, has been isolated, selected and expanded clonally for the following particular metabolic characteristics: rapid cell cycle; doubling every six to eight hours requiring replacement of the culture medium every twelve hours (replacement of 50-70% of the culture medium every twelve hours).

By way of example, said immortal PSC-THP1-EP-FAST cell line, with mature hepatocytic phenotype and rapid metabolic cycle, has the following characterisation in relation to certain cell markers: human HLA-DR1 MHC class II, CD34+, CD44+, CD68+, CD71+, CD81+, CD90+, CD105+, CD117+, TLR5, CDw328, CDw156c, CD172g , LDL Rec (LDL5/6+)+, GATA-4+, HNF-1alfa+, HNF-3beta+, IL-1 RT 1+, CK8+, CK18+, CK7-/+, CK19--/+,c-Met, Alphafetoprotein--/+, ALBUMIN+.

These cells have been induced to differentiate, acquiring the morphology and functional features of mature hepatocytes, by subjecting them in culture to the continuous stimulus generated by the following growth factors: HGF (Hepatocyte Growth Factor), M-CSF (Macrophage Colony-Stimulating Growth Factor), gentamycin, EGF (Epithelial Growth Factor), glucose, a combination of Interleukin 2 and Interleukin 6 and aminoacids.

The cell lines of the invention PSC-THP1-EP, PSC-THP1-EP-FAST, PSC-THP1-HEP and PSC-THP1-EPEP also secrete into the culture supernatant, in addition to albumin, the blood coagulation factors listed in detail in Table 1, for which an activity of between 5% and 80% was detected.

**Table 1.**

| Blood coagulation factors |
|---|
| Antithrombin III |
| Von Willebrand Factor Preproprotein |
| Ser/Cys Proteinase Inhibitor C |
| Urokinase Plasminogen Activator Receptor(UPAR Human) |
| Anticoagulant Slow Form Of Thrombin (THRB Human) |
| Coagulation Factor V |
| Coagulation Factor VII |
| Coagulation Factor VIII |
| Coagulation Factor XIIIB |
| Alpha-2-Macroglobulin |
| Protein C (Inactivator Of Coagulation Factors Va And Viiia) |
| Protein S (Alpha) |
| APC (Activated Protein C Receptor) |
| Alpha-1-Antitrypsin Precursor (Alpha-1 Protease Inhibitor/Alpha-1- |
| Antiproteinase |
| Cl Esterase Inhibitor |

### Cell line

The THP-1 monocytoid cells [16] have been washed three times by centrifugation at 160 g for 10 minutes at room temperature in phosphate buffer (PBS, pH 7.2) and resuspended in 50 ml tubes (Nunc, Kamstrup, Denmark) at a final concentration of 10 X 10⁶ cells/ml, again in phosphate buffer (PBS, pH 7.2).

Samples of all the cells forming the subject of the research have been labelled in vivo with anti-human mouse monoclonal antibodies (Mabs) conjugated to R-phycoerythrin, anti-human CD34 (Santa Cruz Biotechnology, California, USA), anti-human CD14 (Santa Cruz Biotechnology, California, USA), anti-human CD90 (Santa Cruz Biotechnology, California, USA) washed three times by centrifugation at 160 g for 10 minutes at 37°C, and have been subjected to *in vivo* analysis and selection by sorting (Coulter, Hialeath, FL, USA). The cells, isolated by sterile *in vivo* cellular cytofluorimetry (CELL SORTER) have at least the three markers of stem cell expression, believed by ourselves to be fundamental, namely CD34, CD14 and CD90.

The undifferentiated pluripotent stem cells (PSCs-THP1-CD34+eCD14+eCD90+) have been resuspended at a final concentration of 1 X 10⁶ cells per ml in 6 well plates (Lab-Tek chamber slides, Nunc, Kamstrup, Denmark) in 0.5 ml per well of final solution composed of AIM-V medium (GIBCO) supplemented with:
10% FCS (Celbio, Milan, Italy);
100 units/mL penicillin;
100 µg/mL streptomycin;
160 mg/L gentamycin (Schering-Plough, Milan, Italy);
2 mM L-glutamine (Life Technologies; growth medium);
25 ng/mL M-CSF (macrophage colony stimulating factor, Peprotech Inc., NJ, USA).

The cells have been incubated for 10 days in a Heraeus thermostatically controlled incubator at a temperature of 37°C in an atmosphere containing a constant flow of 5% CO₂ (v/v in air) with 0.25 ml of medium being replaced every 3 days.

Upon completion of the incubation period, the cells all show fibroblastoid morphology. The cells harvested using 2% lidocaine (Sigma Aldrich, Milan, Italy) in PBS [17,21] have been washed 3 times by centrifugation at 160 g for 10 minutes at 37°C in RPMI 1640 (Life Technologies, Grand Island, NY) and have been incubated for a second time in accordance with the following description.

The samples destined for stimulation to become specialised hepatocytic cells (PSC-THP1-EP) have been resuspended at a final concentration of 1 X 106 cells per ml in 6 well plates (Lab-Tek chamber slides, Nunc, Kamstrup, Denmark) in 0.5 ml per well of final solution composed of medium described as follows:

**Table 1**

| **REAGENT** | **Quantity/L** |
|---|---|
| AIM-V Medium (Sigma 12055-091) | 700 ml/L |
| F-12 (Gibco 31765) | 200 ml/L |
| L-15 Medium LEIBOVITZ(Sigma L 1518) | 100 ml/L |
| MEM non essential AA(Sigma M 7145) | 10 ml/L |
| HANKS balanced salt sol. (H9269, 500 ml, Sigma) | 4 ml/L |
| Glucose | 10 g/L |
| L-Glutamine (Sigma G 7513) | 2 mM |
| Gentamycin (gentalyn vial 80 mg/2 ml) | 160 mg/L |
| Dexamethasone (vial 4 mg/l ml) | 10⁻⁷M |
| Scopolamine (buscopan vial 20 mg/ml) | 2 mg/L |
| Alpha-nicotinamide (Sigma N6754, 25 mg) | 10 mg/L |
| Beta-nicotinamide (Sigma N1630, 100 mg) | 10 mg/L |
| Glucagon (Glucagen, 1 mg/1 ml, NovoNordisk) | 10⁻⁷M |
| Insulin (I-9278,5 ml Sigma) | 10 mg/L |
| Synthechol NSO (S5442, 10 ml, Sigma) | 4 ml/L |
| Linoleic acid (L1012,100 mg Sigma) | 100 mg/L |
| Transferrin H.(T8158,100 mg Sigma) | 40 mg/L |
| Sodium selenite (S5261,10 g Sigma) | 6.25 µg/L |
| IL-2 (Peprotech) | 25 µg/L |
| IL-6 (Prospec Techno Gene) | 25 µg/L |
| MCSF (Prospec Techno Gene) | 25 µg/L |
| HGF (Prospec Techno Gene) | 25 µg/L |
| EGF (E 9644, 2 mg, Sigma) | 25 µg/L |

Furthermore, as sources of protein, the substances listed in Table 2 are added:

**Table 2**

| | |
|---|---|
| IMA (iron-mannitol-albumin, LDO, VC) | 20 g/L |
| Gamma aminobutyrric acid (GABA) | 2 mM |
| Delta aminolevulinic acid | 0.1% |

After 23 days in culture, the study culture supernatants are collected and stored at -80°C for the following laboratory analyses: the concentrations of microalbumin, calcium, alphafetoprotein, glucose, glycogen and urea.

Again, after 23 days in culture, cell suspensions of all the study samples have been obtained following incubation for 5-8 minutes with 2% lidocaine (Sigma Aldrich, Milan, Italy) in PBS, by pipetting the cell suspension up and down in the well and then harvesting the solution obtained, as described in the bibliography [17, 21].

The cells have been washed three times by centrifugation at 160 g for 10 minutes at 37°C with unsupplemented RPMI 1640 (Life Technologies, Grand Island, NY).

From the cell pellet obtained, a small portion of the cells have been resuspended in 15 ml tubes (Lab-Tek chamber slides, Nunc, Kamstrup, Denmark) at a final concentration of 5 X 10⁵ cells/ml for the subsequent phenotypic analyses (Western Blotting, direct immunofluorescence and FACS) and a portion of the cells have been centrifuged at 160 g for 10 minutes at 4°C in PBS and the cell pellet obtained has been dried completely and immediately frozen at -80°C for subsequent RNA analysis (PCR).

### CELL SUBCLONING

Samples of PSC-THP1-EP cells have been resuspended at a final concentration of 1/3 cells per well in 96 well plates (Lab-Tek chamber slides, Nunc, Kamstrup, Denmark) in 0.05 ml (50 µg) per well of final solution composed of medium described as follows:

**Table 1 bis**

| **REAGENT** | **Quantity/L** |
|---|---|
| AIM-V Medium (Sigma 12055-091) | 900 ml/L |
| F-12 (Gibco 31765) | 20 ml/L |
| L-15 Medium LEIBOVITZ(Sigma L 1518) | 10 ml/L |
| MEM non essential AA(Sigma M 7145) | 1 ml/L |
| HANKS balanced salt sol. (H9269, 500 ml, Sigma) | 0.4 ml/L |
| Glucose | 1 g/L |
| L-Glutamine (Sigma G 7513) | 0.2 mM |
| Gentamycin (gentalyn vial 80 mg/2 ml) | 16 mg/L |
| Dexamethasone (vial 4 mg/1ml) | 10⁻⁸M |
| Scopolamine (buscopan vial 20 mg/ml) | 0.2 mg/L |
| Alpha-nicotinamide (Sigma N6754, 25 mg) | 1 mg/L |
| Beta-nicotinamide (Sigma N1630,100 mg) | 1 mg/L |
| Glucagon (Glucagen, 1 mg/1 ml, NovoNordisk) | 10⁻⁸M |
| Insulin (I-9278, 5 nil Sigma) | 1 mg/L |
| Synthechol NSO (S5442, 10 ml, Sigma) | 0.4 ml/L |
| Linoleic acid (L1012, 100 mg Sigma) | 10 mg/L |
| Transferrin H.(T8158, 100 mg Sigma) | 4 mg/L |
| Sodium selenite (S5261,10 g Sigma) | 0.625 µg/L |
| IL-2 (Peprotech) | 2.5 µg/L |
| IL-6 (Prospec Techno Gene) | 2.5 µg/L |
| MCSF (Prospec Techno Gene) | 2.5 µg/L |
| HGF (Prospec Techno Gene) | 2.5 µg/L |
| EGF (E 9644, 2 mg, Sigma) | 2.5 µg/L |

Furthermore, as sources of protein, the substances listed in Table 2 are added:

**Table 2**

| | |
|---|---|
| IMA (iron-mannitol-albumin, LDO,VC) | 20 g/L |
| Gamma aminobutyrric acid (GABA) | 0.25 mM |
| Delta aminolevulinic acid | 0.1% |

After 23 days in culture, the study culture supernatants are collected and stored at -80°C for the following laboratory analyses: the concentrations of microalbumin, calcium, alphafetoprotein, glucose, glycogen and urea. Following metabolic typing, a cellular subclone with a particularly rapid metabolic cycle has been selected from the PSC-THP1-EP line (known as PSC-THP1-EP-FAST) and has been expanded.

### CELL FUSION

The genetic information contained in cells of various origins may be combined in a single nucleus by means of cell fusion. Cell fusion requires that the cells come into contact, and includes brief destruction of the cell membranes using chemical agents. When membrane reconstruction occurs, adjacent cells can reform their membranes together, thus producing a single hybrid cell. Initially, fusion derived cells contain two/four nuclei (polynucleated heterokaryons), but following cellular division, the chromosomal complement of the two cells becomes packaged into a single nucleus (synkaryons).

The cell fusion technique uses PEG (polyethyleneglycol).

### CELL FUSION METHOD

Samples of two million cells are harvested for each cell type considered for cell fusion (namely, for PSC-THP1-HEP: HEPG2 cells and PSC-THP1-EP cells; for PSC-THP1-EPEP: HEPG2 cells, PSC-THP1-EP cells and primary human hepatocytes) of two or more human cell types (leukaemic or primary) in suspension or adhered. Adhered cells are detached in a specific manner depending on cell type, as described above. All the cells considered for fusion are collected in a single 50 ml tube (Lab-Tek chamber slides, Nunc, Kamstrup, Denmark) and washed twice by centrifugation at 160 g for 7 minutes at 37°C in serum-free DMEM-LG medium (Gibco; Grand Island, NY). The cells obtained by pelleting are resuspended by repeated pipetting up and down in 2 ml of PEG for every 4 million cells, for 60 seconds. After 60 seconds of pipetting, 10 ml of FCS or FBS are added and the cells washed by centrifugation at 160 g for 7 minutes at 37°C. The cells thus obtained are washed twice by centrifugation at 160 g for 7 minutes at 37°C in DMEM-LG medium (Gibco; Grand Island, NY) with 20% serum (FCS or FBS) and resuspended at a concentration of 1X10⁶ cells/ml in AIM-V medium supplemented with 20% FCS and incubated for 3 days in a Heraeus thermostatically controlled incubator at a temperature of 37°C, with an atmosphere containing a constant 5% CO₂ (v/v in air).

On completion of incubation, the cells thus obtained (PSC-THP1-HEP or PSC-THP1-EPEP) have been washed three times by centrifugation at 160 g for 10 minutes at 37°C in RPMI 1640 (Life Technologies, Grand Island, NY) and have then been placed in culture in 25 cm² flasks (Nunc, Kamstrup, Denmark) at a final concentration of 1X10⁶ cells/ml in a final solution composed of the medium described below:

**Table 1**

| **REAGENT** | **Quantity/L** |
|---|---|
| AIM-V Medium (Sigma 12055-091) | 700 ml/L |
| F-12 (Gibco 31765) | 200 ml/L |
| L-15 Medium LEIBOVITZ(Sigma L 1518) | 100 ml/L |
| MEM non essential AA(Sigma M 7145) | 10 ml/L |
| HANKS balanced salt sol. (H9269, 500 ml, Sigma) | 4 ml/L |
| Glucose | 10 g/L |
| L-Glutamine (Sigma G 7513) | 2 mM |
| Gentamycin (gentalyn vial 80 mg/2 ml) | 160 mg/L |
| Dexamethasone (vial 4 mg/1 ml) | 10⁻⁷M |
| Scopolamine (buscopan vial 20 mg/ml) | 2 mg/L |
| Alpha-nicotinamide (Sigma N6754, 25 mg) | 10 mg/L |
| Beta-nicotinamide (Sigma N1630, 100 mg) | 10 mg/L |
| Glucagon (Glucagen, 1 mg/1 ml, NovoNordisk) | 10⁻⁷M |
| Insulin (I-9278, 5 ml Sigma) | 10 mg/L |
| Synthechol NSO (S5442, 10 ml, Sigma) | 4 ml/L |
| Linoleic acid (L1012,100 mg Sigma) | 100 mg/L |
| Transferrin H.(T8158, 100 mg Sigma) | 40 mg/L |
| Sodium selenite (S5261,10 g Sigma) | 6.25 µg/L |
| IL-2 (Peprotech) | 25 µg/L |
| IL-6 (Prospec Techno Gene) | 25 µg/L |
| MCSF (Prospec Techno Gene) | 25 µg/L |
| HGF (Prospec Techno Gene) | 25 µg/L |
| EGF (E 9644, 2 mg, Sigma) | 25 µg/L |

Furthermore, as sources of protein, the substances listed in Table 2 are added:

**Table 2**

| | |
|---|---|
| IMA (iron-mannitol-albumin, LDO, VC) | 20 g/L |
| Gamma aminobutyrric acid (GABA) | 2 mM |
| Delta aminolevulinic acid | 0.1% |

### CELL FUSION: Light microscopy

After being washed three times by centrifugation at 160 g for 10 minutes at room temperature in PBS (pH 7.4), the cell pellets are seeded onto slides and the liquid allowed to evaporate in the air. The samples are fixed in 95% ethanol for 15 minutes at room temperature. After gentle rinsing three times in PBS for 30 seconds each, the cells have been treated with a solution of haematoxylin (Sigma Aldrich) for 5 minutes; the slides remain upturned in stain in an appropriate container known as a "watch glass" with the purpose of avoiding any precipitation onto the slices. This is followed be gentle rinsing in PBS for approx. 10 minutes; during this stage, conducted for the purpose of stabilising the stain, the colour of the stain develops from burgundy red to blue, stabilising the bonds. Cells are treated with an eosin solution (Sigma Aldrich Inc.) for 1 minute; again in this case, the slides remain upturned in stain in a suitable container known as a "watch glass" with the purpose of avoiding any precipitation onto the slices. This is followed by gentle rinsing in PBS for approx. 10 seconds then rapid dehydration in 99% ethanol. Finally, a drop of hot (56°C) moviol (Vectashield) is placed in the centre of the slide in question and cover-slides mounted. The preparations are observed by light microscopy (100X), assessing the morphology of the cellular nuclei and cytoplasm [10-11].

### CELL FUSION: Fluorescence microscopy

The cell pellets have been resuspended in a fixing solution of 4% paraformaldehyde in RPMI 1640 at pH 7.4, for one hour at room temperature. After washing three times in PBS, the cells have been resuspended in a solution of PBS and 0.1% Triton for one hour at 4°C. After washing three times in PBS, the cells have been seeded onto slide covers and the liquid evaporated off in air; Hoechst solution is then added (DAPI, 1:1000 dilution), a stain that stains cell nuclei blue by binding to the DNA double helix, and left for 1 minute. The final 3 washes are performed and the cover slips with the cells are mounted onto slides using Moviol (Vectashield), an adhesive which maintains the fluorescence for approx. one month. After one night at 4°C, the cells are ready for observation by fluorescence microscopy.

The cells belonging to the samples subjected to cell fusion, had a significantly high percentage of cell fusion (90% of the sample compared to 0% of the untreated controls).

Karyotypic analysis of the sample cells three months after fusion showed 95% tetraploidy, compared to 0% of the untreated controls.

Naturally, the details and forms of embodiment may be altered extensively with respect to the details described and illustrated without departing from the scope of protection of the present invention, as defined in the appended claims.

### Western blotting

The samples have been subjected to phenotypic analysis by Western Blotting for markers using anti-CD 14 (Santa Cruz Biotechnology, California, USA), anti-CD 29 (Santa Cruz Biotechnology, California, USA), anti-CD 34 (Santa Cruz Biotechnology, California, USA), anti-CD 44 (Santa Cruz Biotechnology, California, USA), anti-CD 45 (Santa Cruz Biotechnology, California, USA), anti-CD 71 (Santa Cruz Biotechnology, California, USA), anti-CD 90 (Santa Cruz Biotechnology, California, USA), anti-CD 105 (Santa Cruz Biotechnology, California, USA), anti CD 117/c-KIT (Santa Cruz Biotechnology, California, USA), anti c-MET (Santa Cruz Biotechnology, California, USA), anti cytokeratin 7 (Santa Cruz Biotechnology, California, USA), anti cytokeratin 8 (Santa Cruz Biotechnology, California, USA), anti cytokeratin 18 (Santa Cruz Biotechnology, California, USA), anti cytokeratin 19 (Santa Cruz Biotechnology, California, USA), anti cytokeratins 7/17 (Santa Cruz Biotechnology, California, USA), anti albumin (Rockland Immunochemicals, Pennsylvania, USA), anti alphafetoprotein (Monosan Europa, Netherlands). After five washes, the membranes have been incubated with the corresponding secondary antibodies (1:1000) conjugated to horseradish peroxidase (HRP, SantaCruz Biotechnologies Inc., Santa Cruz, CA, USA) for 1 hour at room temperature, as reported in the following tables.

### Immunofluorescence protocol

Cells in suspension have been incubated with 0.2 mM MitoTracker Red for 10 minutes at 37°C. After three washes by centrifugation at 160 g for 10 minutes at room temperature in PBS (pH 7.4), the cell pellets have been resuspended in a fixing solution of 4% paraformaldehyde in RPMI 1640 at pH 7.4, 1 hour at room temperature. After three washes in PBS, the cells have been resuspended in a solution of PBS and 0.1% Triton for 1 hour at 4°C. After three washes in PBS, the cells have been seeded onto slide covers and the liquid allowed to evaporate-off in air. The cells have been blocked with 20% normal goat serum for one hour, and then incubated for 30 minutes with the following anti-human monoclonal antibodies: anti-CD 14 (Santa Cruz Biotechnology, California, USA), anti-CD 29 (Santa Cruz Biotechnology, California, USA), anti-CD 34 (Santa Cruz Biotechnology, California, USA), anti-CD 44 (Santa Cruz Biotechnology, California, USA), anti-CD 45 (Santa Cruz Biotechnology, California, USA), anti-CD 71 (Santa Cruz Biotechnology, California, USA), anti-CD 90 (Santa Cruz Biotechnology, California, USA), anti-CD 105 (Santa Cruz Biotechnology, California, USA), anti CD 117/c-KIT (Santa Cruz Biotechnology, California, USA), anti c-MET (Santa Cruz Biotechnology, California, USA), anti cytokeratin 7 (Santa Cruz Biotechnology, California, USA), anti cytokeratin 8 (Santa Cruz Biotechnology, California, USA), anti cytokeratin 18 (Santa Cruz Biotechnology, California, USA), anti cytokeratin 19 (Santa Cruz Biotechnology, California, USA), anti cytokeratins 7/17 (Santa Cruz Biotechnology, California, USA), anti albumin (Rockland Immunochemicals, Pennsylvania, USA), anti alphafetoprotein (Monosan Europa, Netherlands) conjugated to phycoerythrin (PE) or fluorescein isothiocyanate (FITC). Specific controls with the corresponding isotypes have been devised for each monoclonal antibody (Santa Cruz Biotechnology, CA, USA). The nuclei have been stained using Hoechst solution (dilution 1:1000). The cover slips, mounted onto slides using moviol have been examined by light microscopy [16-17].

### Flow cytofluorometric analysis (FACS)

The cells have been transferred into 50 ml tubes (Lab-Tek chamber slides, Nunc, Kamstrup, Denmark) and washed three times by centrifugation at 160 g for 10 minutes at room temperature in PBS (pH 7.4). The pellets have been resuspended at a final concentration of 5X10⁵ cells/ml in PBS. The cells in suspension have been incubated with 0.2 mM MitoTracker Red for 10 minutes at 37°C. After washing three times with PBS the cells have been fixed for one hour in 4% paraformaldehyde in PBS (pH 7.4) at room temperature. After washing three times in PBS, the cells have been resuspended in a solution of PBS and 0.1% Triton for one hour at 4°C. After washing five times in PBS, the cells have been resuspended in a blocking solution of 20% normal goat serum for one hour. After washing three times in PBS, the samples have been incubated for 30 minutes with the following antihuman monoclonal antibodies: anti-CD 14 (Santa Cruz Biotechnology, California, USA), anti-CD 29 (Santa Cruz Biotechnology, California, USA), anti-CD 34 (Santa Cruz Biotechnology, California, USA), anti-CD 44 (Santa Cruz Biotechnology, California, USA), anti-CD 45 (Santa Cruz Biotechnology, California, USA), anti-CD 71 (Santa Cruz Biotechnology, California, USA), anti-CD 90 (Santa Cruz Biotechnology, California, USA), anti-CD 105 (Santa Cruz Biotechnology, California, USA), anti CD 117/c-KIT (Santa Cruz Biotechnology, California, USA), anti c-MET (Santa Cruz Biotechnology, California, USA), anti cytokeratin 7 (Santa Cruz Biotechnology, California, USA), anti cytokeratin 8 (Santa Cruz Biotechnology, California, USA), anti cytokeratin 18 (Santa Cruz Biotechnology, California, USA), anti cytokeratin 19 (Santa Cruz Biotechnology, California, USA), anti cytokeratins 7/17 (Santa Cruz Biotechnology, California, USA), anti albumin (Rockland Immunochemicals, Pennsylvania, USA), anti alphafetoprotein (Monosan Europa, Netherlands). All the antibodies used are monoclonal, conjugated to R-phycoerythrin (PE) or Fluorescein-IsoThioCyanate (FITC). Specific controls with the corresponding isotypes have been devised for each monoclonal antibody (Santa Cruz Biotechnology, California, USA). The samples have been subjected to quantitative analysis using a laser cytofluorimeter (Epics Profile II, Coulter, Hialeath, FL) at 488 nm and referred to the percentage of fluorescent cells (PFC) as the geometric mean. Threshold values (gates) have been established using control samples labelled with the corresponding isotypes. All values have been analysed with a minimum threshold of 15,000 cells.

### RNA-PCR (RNA-Polymerase Chain Reaction)

### RNA extraction

RNA extraction has been performed using Tri Reagent solution (Molecular Research Center, Inc.) in accordance with the manufacturer's instructions. The cell pellets have been resuspended in 1 ml of Tri Reagent solution per sample and incubated for 5 minutes at room temperature. Following incubation, 100 µl of bromochloropropane have been added and the samples incubated again for 15 minutes at room temperature. After centrifugation at 12000 g for 15 minutes at 4°C, the aqueous phases have been recovered and transferred into Eppendorf type tubes (approx. 500 µl), along with 500 µl of isopropanol. The samples have been incubated for 10 minutes at room temperature, then centrifuged at 12000 g for 8 minutes at 4°C and washed with 1 ml of 75% ethanol, then centrifuged at 7500 g for 5 minutes and finally the supernatant removed using a syringe and the pellets carefully dried under a fume hood. Finally, the RNA samples have been resuspended in 50 µl of DNAse-free water.

### Spectrophotometric measurement

Resuspend in 50 µl of immediately opened, autoclaved MilliQ H₂O, read 5 µl using the spectrophotometer (dilution: 1:100).

### RT-PCR

Messenger RNAs have been Retro-Transcribed using the Applied Biosystems "cDNA Archive Kit" kit (Applied Biosystems). Reactions have been prepared in a total volume of 50 µl, with 10 µl of sample solution + H₂0 in the presence of 0.25 IU of RNAse (Promega). The samples have been incubated at 25°C for 10 minutes and then at 37°C for 2 hours.

**Table 3.**

| | |
|---|---|
| Sample + H₂0 | 10 µl |
| 10x buffer | 5 µl |
| 25x dNTPs | 2 µl |
| 10x random primers | 5 µl |
| multi scribe RT | 25 µl |
| RNAsin | 0.25 U |

The samples have been inserted into the PCR machine for 10 minutes at 25°C and 2 hours at 37°C. The cDNAs have been stored at -20°C.

### Removal of genomic DNA

Genomic DNA has been removed using the "TURBO DNA-free" system (Ambion), by incubation for 30 minutes at 37°C. The DNAse has been removed by incubating each sample with 5 µl DNAse removing agent, for 2 minutes a room temperature. The samples have then been centrifuged at 10,000xg for 1 minute, and finally, 1 µl of each reverse transcribed sample used for Real Time PCR amplification.

**Table 4.**

| | |
|---|---|
| RNA | 50 µl |
| DNAse buffer | 5 µl |
| DNAse | 2 µl |

The following primers have been used for Real Time PCR amplification, performed using the "SYBR Green" method (SYBR Green Master Mix kit, Applied Biosystems):
ALB_fw 5' - GAGATGCACACAAGAGTGAG - 3'
ALB_rv 5' - CTGAGCAAAGGCAATCAACA - 3'
AFP_fw 5' - GGGAGCGGCTGACATTATTA - 3'
AFP_rv 5' - TCTTGCTTCATCGTTTGCAG - 3'
MET_fw 5' - CTCCTCTGGGAGCTGATGAC - 3'
MET_rv 5' - GGTGCCAGCATTTTAGCATT - 3'
GAPDH_fw 5' - GAAGGTGAAGGTCGGAGTC - 3'
GAPDH_rv 5' - GAAGATGGTGATGGGATTTC - 3'

The following concentrations of *primers* (nM) have been tested for the purpose of amplifying the reaction (both directions): ALB 50/150; AFP 50/300; MET 300/300; GAPDH 50/300.

The reactions have been diluted by adjusting the solutions to 100 µl with H₂O and 1 µl used for each amplification reaction. The reactions have been prepared using the "SYBR Green Master Mix" (Applied Biosystems) kit, in a total volume of 25 µl. The reactions have been performed in triplicate. Amplification reactions have been performed using an "ABI Prism 7700 Sequence Detector" (Perkin Elmer). The data has been analysed using Sequence Detection 1.9.1 software (Perkin Elmer).

### Levels of alphafetoprotein, urea albumin and microalbumin in the cell culture supernatants tested

All measurements have been performed using a Modular Analytics P automated system (Roche-Hitachi).

### Identification of coagulation factors in the cell culture supernatants tested

All measurements have been performed using a MALDI-TOF-MS (Micro MX, Waters, Manchester, UK) automated system. The peptides identified have been processed using the ProFound-Peptide Mapping on-line search engine for the reconstruction of the sequences and the corresponding proteins using the PMF, Peptide Mass Fingerprinting technique (EMBL-EBI, European Bioinformatic Institute; NCBI, National Center for Biotechnology Information).

### EXAMPLE 1. Light microscopy

Following sorting and expansion of the selected cell clones, at 10 days of incubation the samples showed the morphological transformation of the THP1 cells, from round cells to an elongated fibroblastoid shape adhering to the culture plate.

The controls showed a purely rounded shape and were entirely in suspension.

### EXAMPLE 2. Light microscopy: incubation for 23 days

After 23 days of incubation in AIM-V medium, supplemented as described previously, the samples showed the morphological transformation of the THP1 cells from rounded to a tetrahedral shape adhered to the culture plate, positive for the production of: albumin, alphafetoprotein, glycogen, cytokeratin 7, 8, 17, 18 and 19, OV-6, c-Met receptor (FACS, immunohistochemistry, WB, PCR) and several blood coagulation factors (Western Blotting, MALDI-TOF-MS, 2D electrophoresis), as listed in Table 1.

The controls only showed rounded morphology and were all in suspension with negative results for albumin, alpha-fetoprotein, glycogen, cytokeratin 7, 8, 17, 18 and 19, OV-6.

### EXAMPLE 3. Characterisation of THP-1 versus PSCs-THP-CD34+/CD14+/CD90+ and PSCs-THP1-EP and PSC-THP1-EP-FAST

The results pertaining to the expression of CD14+, CD29+, CD34+, CD44+, CD45-/+, CD71+, CD90+, CD105+, CD117+, c-Met, cytokeratin 7, cytokeratin 8, cytokeratin 18, cytokeratin 19, cytokeratins 7/17 have been expressed on a quantitative scale, as shown in Table 5 below.

**Table 5**

| Surface antigens | THP-1 | PSCs-THP1-CD34+/CD14+ /CD90+ | PSC-THP1-EP PSC-THP1-EP-FAST |
|---|---|---|---|
| | | | Sample |
| *CD14* | ++ | ++++ | + |
| *CD29* | + | ++ | ----- |
| *CD34* | +++ | +++++ | ----- |
| *CD44* | ++ | +++ | ----- |
| *CD45* | ++++ | + | ----- |
| *CD71* | ++ | ++++ | +++ |
| *CD90* | + | +++++ | ----- |
| *CD105* | ++ | +++ | ----- |
| *CD117* | ++ | +++ | + |
| *c-MET* | +++ | +++ | ++ |
| *Cytokeratin 7* | + | ++ | +++++ |
| *Cytokeratin 8* | + | ++ | ++++ |
| *Cytokeratin 18* | ++ | +++ | +++ |
| *Cytokeratin 19* | ++ | +++ | +++++ |
| *Cytokeratins 7*/*17* | ++ | ++ | ++++ |

| | | | |
|---|---|---|---|
| ----- = absence of any fluorescence + =1-5 fluorescent cells per optical field ++ = 6-10 fluorescent cells per optical field +++ = 10-20 fluorescent cells per optical field ++++ = 20-50 fluorescent cells per optical field +++++ > 50 fluorescent cells per optical field | | | |

### EXAMPLE 4. Western Blotting

The cells have been subjected to phenotypic analysis by Western Blotting for the markers albumin and alphafetoprotein, as discussed below.

The quantitative indications reported in the following tables must be read according to the following keys:
--- = absence of any bands
-/+ = slight presence of a band
+ = thin band present
++ = medium band present
+++ = broad band present
++++ = high band present
+++++ = abundant band present

### 1. ALBUMIN (Rockland Immunochemicals, Pennsylvania, USA)

The results show slight positivity for the production of human albumin in control THP-1 cells, subtle positivity in PSC-THP1 CD34+eCD14+eCD90+ cells, as opposed to marked positivity for the production of human albumin in HEPG2 cells, and extensive positivity in PSC-THP1-EP, PSC-THP1-EP-FAST, PSC-THP1-HEP, PSC-THP1-EPEP cells and for human albumin, as reported in Table 6.

**Table 6.**

| **Anti human albumin monoclonal antibody** | | | | | |
|---|---|---|---|---|---|
| Medium | HepG2 | THP-1 | PSCs-THP1-CD34+/CD1 4+/CD90+ | PSC-THP1-EP PSC-THP1-EP-FAST PSC-THP1-HEP PSC-THP1-EPEP Sample | Human albumin |
| --- | ++++ | -/+ | + | +++++ | +++++ |

### 2. ALPHAFETOPROTEIN (Monosan Europa, Netherlands), Staining with anti-alphafetoprotein HRP Mabs gave the results in Table 7 below.

**Table 7.**

| Anti human alphafetoprotein monoclonal antibody | | | |
|---|---|---|---|
| Medium | THP-1 | PSC-THP1-EP PSC-THP1-EP-FAST PSC-THP1-EPEP | PSC-THP1-HEP HepG2 |
| | | Sample | |
| --- | --- | -/+ | +++++ |

### EXAMPLE 5. Characterisation of THP-1 versus PSCs-THP-CD34+/CD14+/CD90+ and PSCs-THP1-EP cells and PSC-THP1-EP-FAST by cytofluorimetry (FACS)

Adult blood contains haematopoietic progenitor cells. Among the haematopoietic cells, the so-called "HSC" cells (CD14+ and CD34+) are pluripotent cells (Zhao, 2003) and, when suitably stimulated by growth factors, can differentiate and specialise into cells typical of various body parts, including the liver [1-2].

Cultured THP-1 cells have been selected by sorting for high fluorescence positivity for CD14+, CD34+ and CD90+; said selected cell clones were isolated in culture and expanded, and, after ten days, it was possible to observe how said fluorescence remained stable in culture. Untreated controls showed slight fluorescence for CD29+, CD34+, CD44+, CD90+, CD105+.

THP-1 cells incubated in culture with 25 ng/mL HGF for 50 days show no fluorescence for CD14+, CD29+, CD34+, CD44+, CD45-/+, CD90+, CD105+ (PSCs-THP1), but are slightly positive for c-Met and CD117 and show high fluorescence for albumin, alphafetoprotein and cytokeratins (7, 17, 8, 18 and 19) and CD71. Untreated controls showed fluorescence for CD14 and CD45. The results are reported in Tables 8 and 9 as the geometric mean +/- the Standard Deviation (SD).

**Table 8.**

| Surface antigens | THP-1 | PSCs-THP1-CD34+/CD1 4+/CD90+ | PSC-THP1-EP PSC-THP1-EP-FAST |
|---|---|---|---|
| | | | Sample |
| *CD14* | 142 +/- 5 | 213 +/- 25 | 7 +/- 6 |
| *CD29* | 2 +/- 2 | 20 +/- 8 | 2 +/- 2 |
| *CD44* | 3 +/- 1 | 15+/-5 | 9 +/- 6 |
| *CD34* | 12+/-5 | 220 +/- 9 | 9 +/- 6 |
| *CD45* | 26 +/- 6 | 19 +/- 4 | 7 +/- 3 |
| *CD71* | 23 +/- 1 | 38 +/- 5 | 59 +/- 30 |
| *CD90* | 7+/-5 | 222 +/- 8 | 8 +/- 6 |
| *CD105* | 3 +/- 1 | 28+/-7 | 2 +/- 2 |
| *CD117* | 36 +/- 2 | 38 +/- 4 | 4 +/- 2 |
| *c-Met* | 6 +/- 2 | 32 +/- 9 | 28 +/- 6 |

**Table 9.**

| Antigens Tested | THP-1 | PSCs-THP1-CD34+/CD1 4+/CD90+ | PSC-THP1-EP PSC-THP1-EP-FAST |
|---|---|---|---|
| | | | Sample |
| Albumin | 18 +/- 4 | 18+/-10 | 90+/-20 |
| alphafetoprotein | 10 +/- 8 | 3 +/-1 | 18 +/- 12 |
| Cytokeratin 7 | 7+/-1 | 19+/-3 | 80 +/- 25 |
| Cytokeratin 8 | 5 +/- 2 | 22 +/-3 | 85 +/- 53 |
| Cytokeratin 18 | 3 +/-1 | 18 +/- 5 | 59 +/- 30 |
| Cytokeratin 19 | 6+/- 2 | 25 +/-5 | 72 +/- 24 |

### Albumin

Cell samples in culture, treated with HGF at various incubation times (most representative times - 7, 15, 23 days) have been analysed.

In accordance with the previous analyses, the data shows the production of albumin by mature hepatocytic-like cells (PSC-THP1-EP) derived from the THP-1 cell line selected by pluripotent stem cell sorting (PSC-THP1-CD34+/CD14+/CD90+).

The data relating to microalbumin in the samples tested confirm the previous data on albumin, both numerically and temporally. Indeed, concentrations of microalbumin above the minimum detectable limits are observed in only 3 samples treated with HGF (PSC-THP1-EP) sampled at day 7, 15 and 30 of the experiment.

### Untreated THP-1 control cells.

At all incubation times, the THP-1 control cells show no significant presence of albumin by cytofluorimetry. By way of example, the data relating to the controls at day 23 are shown.

### THP-1 cells treated with MCSF (PSCs-THP1).

At all incubation times, the THP-1 cells treated with 25 ng/mL MCSF (PSC-THP1) show no detectable albumin by cytofluorimetry. By way of example, the data relating to the controls at day 23 are shown.

### PSCs-THP1 cells incubated with HGF at 25 ng/mL (7, 15, 23 days).

At low concentrations of growth factors, including HGF (2.5 ng/mL and 25 ng/mL), the differentiation of PSCs-THP1 cells to hepatocytic cells is slow and the appearance of mature hepatocytes, producing a high concentration of albumin, is observed.

### Alphafetoprotein

Samples of untreated THP-1 cells (control) in culture, treated with 50 ng/mL MCSF (PSC-THP1) and treated with various concentrations of (25 ng/mL and 2.5 ng/mL) growth factors (PSC-THP1-EP, PSC-THP1-EP-FAST, PSC-THP1-HEP and PSC-THP1-EPEP) have been analysed at various incubation times (days 7, 15 and 23). A profile similar to that for HEPG2 cells and PSC-THP1-HEP cells (mixed population of hepatocytes and hepatoblasts) with regard to alphafetoprotein production is observed.

At all incubation times, the THP-1 control cells show no significant production of alphafetoprotein by cytofluorimetry. By way of example, the data relating to the controls at day 23 are shown.

### EXAMPLE 6. Real time PCR

### GAPDH

The data show that the mRNA for GAPDH is expressed uniformly in all samples tested, ensuring the correct storage of the mRNA extracted, and the uniformity in the initial concentrations of the samples examined.

### Albumin

The data show that the presence of the mRNA for albumin is only clear in the HepG2 positive control and in the THP1-PSC liver like cells following stimulation with HGF (optimal treatment range: 25 ng/mL HGF) for at least 30 days; in all other controls there appears to be no albumin mRNA.

### Alphafetoprotein

The data shows that the presence of mRNA for alphafetoprotein is only clear in the HepG2 positive control.

### c-Met

The data show the presence of c-Met mRNA is reasonably well expressed in all cells tested. The c-Met mRNA does not appear to be altered by any treatment.

### MALDI-TOF-MS

The PSC-THP1-EP cell culture supernatants have been treated using the method for Western Blotting, and the bands obtained excised and digested. Numerous peptides have been identified from the digestion. The peptides obtained have been processed using the ProFound-Peptide Mapping on-line search engine for reconstruction of the sequences and the identification of the corresponding proteins using the PMF, Peptide Mass Fingerprinting, technique.

The sequences relating to several of the proteins identified in the PSC-THP1-EP, PSC-THP1-EP-FAST, PSC-THP1-HEP and PSC-THP1-EPEP cell culture supernatants, pertaining to the characterisation of the products of said cultures with a mature hepatocytic phenotype, are attached below.

### Albumin.

| Description | ALBUMIN Precursor |
|---|---|
| Molecular weight | 69 kDa |
| Amino acid sequence | 609 AA |
| | MKWVTFISLL FLFSSAYSRG VFRRDAHKSE VAHRFKDLGE |
| | |
| | |
| | |
| | |
| | |
| | |
| | |
| | |
| | |
| | |
| Description | ALBUMIN |
| Molecular weight | 66.4 kDa |
| Amino acid sequence | 585 AA |
| | 1 DAHKSEVAHR FKDLGEENFK ALVLIAFAQY LQQCPFEDHV KLVNEVTEFA KTCVADESAE |
| | 61 NCDKSLHTLF GDKLCTVATL RETYGEMADC |
| | CAKQEPERNE CFLQHKDDNP NLPRLVRPEV |
| | 121 DVMCTAFHDN EETFLKKYLY EIARRHPYFY APELLFFAKR YKAAFTECCQ AADKAACLLP |
| | 181 KLDELRDEGK ASSAKQRLKC ASLQKFGERA FKAWAVAPLS QRFPKAEFAE VSKLVTDLTK |
| | 241 VHTECCHGDL LECADDRADL AKYICENQDS ISSKLKECCE KPLLEKSHCI AEVENDEMPA |
| | 301 DLPSLAADFV ESKDVCKNYA EAKDVFLGMF LYEYARRHPD YSVVLLLRLA KTYETTLEKC |
| | 361 CAAADPHECY AKVFDEFKPL VEEPQNLIKQ NCELFEQLGE YKFQNALLVR YTKKVPQVST |
| | 421 PTLVEVSRNL GKVGSKCCKH PEAKRMPCAE DYLSVVLNQL CVLHEKTPVS DRVTKCCTES |
| | 481 LVNRRPCFSA LEVDETYVPK EFNAETFTFH ADICTLSEKE RQIKKQTALV ELVKHKPKAT |
| | 541 KEQLKAVMDD FAAFVEKCCK ADDKETCFAE EGKKLVAASQ AALGL |

| Blood coagulation factors | |
|---|---|
| Antithrombin III | |
| Von Willebrand Factor Preproprotein | |
| Ser/Cys Proteinase Inhibitor C | |
| Urokinase Plasminogen Activator Receptor(UPAR Human) | |
| Anticoagulant Slow Form Of Thrombin (THRB Human) | |
| Coagulation Factor V | |
| Coagulation Factor VII | |
| Coagulation Factor VIII | |
| Coagulation Factor XIIIB | |
| Alpha-2-Macroglobulin | |
| Protein C (Inactivator Of Coagulation Factors Va And Viiia) | |
| Protein S (Alpha) | |
| APC (Activated Protein C Receptor) | |
| Alpha-1-Antitrypsin Precursor (Alpha-1 Protease Inhibitor/Alpha-1-Antiproteinase | |
| C1 Esterase Inhibitor | |

### Cytochrome P450 (CYP450)

Cytochrome P-450 is a superfamily of enzymes that metabolize a large number of drugs, xenobiotics and endogenous substances in vitro and in vivo. Enzymes of the cytochrome P450 superfamily catalyze the oxidative metabolism of a variety of substrates, including natural compounds such as steroids, fatty acids, prostaglandins, leukotrienes, and vitamins, as well as drugs, carcinogens, mutagens, and xenobiotics. Cytochrome P450s, also known as P450 heme-thiolate proteins, usually act as terminal oxidases in multi-component electron transfer chains, called P450-containing monooxygenase systems. Specific reactions catalyzed include hydroxylation, epoxidation, N-oxidation, sulfooxidation, N-, S-, and O-dealkylations, desulfation, deamination, and reduction of azo, vitro, and N-oxide groups. These reactions are involved in steroidogenesis of glucocorticoids, cortisols, estrogens, and androgens in animals; insecticide resistance in insects; herbicide resistance and flower coloring in plants; and environmental bioremediation by microorganisms. Cytochrome P450 actions on drugs, carcinogens, mutagens, and xenobiotics can result in detoxification or in conversion of the substance to a more toxic product. Cytochrome P450s are abundant in the liver, but also occur in other tissues. Members of the cytochrome P450 family are present in varying levels and their expression and activities are controlled by variables such as chemical environment, sex, developmental stage, nutrition and age.

More than 200 cytochrome P450 genes have been identified. There are multiple forms of these P450 and each of the individual forms exhibit degrees of specificity towards individual chemicals in the above classes of compounds. In some cases, a substrate, whether a drug or a carcinogen, is metabolized by more then one of the cytochromes P450. All cytochrome P450s use a heme cofactor and share structural attributes. Most cytochrome P450s are 400 to 530 amino acids in length. The secondary structure of the enzyme is about 70% alpha-helical and about 22% beta-sheet. Genetic polymorphisms of cytochromes P450 result in phenotypically-distinct subpopulations that differ in their ability to perform biotransformations of particular drugs and other chemical compounds. These phenotypic distinctions have important implications for selection of drugs. For example, a drug that is safe when administered to most humans may cause toxic side-effects in an individual suffering from a defect in an enzyme required for detoxification of the drug. Alternatively, a drug that is effective in most humans may be ineffective in a particular subpopulation because of lack of a enzyme required for conversion of the drug to a metabolically active form. Further, individuals lacking a biotransformation enzyme are often susceptible to cancers from environmental chemicals due to inability to detoxify the chemicals.

The major P450 forms in the human liver are P450 1A2 (CYP 1A2) and P-450 3A (CYP 3A).

Human cytochrome P450 1A2 constitutes about 13% of total P450 in human liver and is the second most abundant P450 following human cytochrome P450 3A4. P450 1A2 catalyzes the metabolism of a large variety of drugs and carcinogens. Drugs metabolized by human P450 1A2 include phenacetin, R-warfarin, clomipramine, imipramine, theophyline, theobromine, paraxanthine, caffeine, chlorzoxazone, 7-methoxyresorufin, and 7-ethoxycoumarin. P450 1A2 also has a major role in activating mutagens and carcinogens. For example, 1A2 metabolically activates the food pyrolysis products IQ and MeIQx to active mutagens. A complication in patient drug choice is that most drugs have not been characterized for their metabolism by P450 1 A2 and other cytochromes P450. Without knowing which cytochrome(s) p450 is/are responsible for metabolizing an individual drug, an assessment cannot be made for the adequacy of a patient's P450 profile. For such drugs, there is a risk of adverse effects if the drugs are administered to deficient metabolizers.

The cytochrome P-450 3A (CYP 3A) isoenzyme is a member of the cytochrome P-450 superfamily. It constitutes up to 60% of the total human liver microsomal cytochrome P-450 and is responsible for metabolism of a large number of drugs including nifedipine, macrofide antibiotics including erythromycin and tri-oleandomycin, cyclosporin A, FK506, teffenadine, tamoxifen, lidocaine, midazolam, triazolam, dapsone, diltiazem, lovastatin, quinidine, ethylestradiol, testosterone, and alfentanil. In addition, CYP 3A has been shown to be involved in both bioactivation and detoxication pathways for several carcinogens in vitro. The active form of CYP 3A has been found in other organs besides the liver including kidney epithelial cells, jejunal mucosa, and the lungs. In these organs, the amount of the cytochrome P450 protein is much lower then in the liver. The presence of the cytochrome P-450 3A in the lung microsomes indicates that the drugs and other substances which are subject to CYP 3A (P450-3A) mediated metabolism may be partially metabolized in the lungs. This has been demonstrated for the topical steroid, beclomethasome dipropionate. It has also been shown that only about 10% of the drug released by an inhaler is available to the lungs. The remaining mount is retained in the spacer device and oral cavity. Steroids absorbed from the lungs and gastrointestinal tract are subsequently metabolized by hepatic cytochrome P450. Many drugs, such as prednisone, cyclosporin, cyclophosphamide, digitoxin, diazepam, ethinylestradiol, midazolam, triazolo-benzodiazepines,dihydropyridine calcium channel blockers, certain HMG-CoA reductase inhibitors, etc. are metabolized by a member of the CYP3A family, CYP3A4.

### Cytochrome P450 and human hepatocytes,

Cytochrome P450 (CYP450) isoforms 1A2, 2A6, 2C9, 2C19, 2D6, 2E1, and 3A4 in cryopreserved human hepatocytes were studied [28, 29]. The method is particularly useful in determining drug-drug interactions [28,29].

Human CYP2A6 is an important member of the CYP superfamily and is present in liver up to 1% of the total CYP content. Human CYP2A6 metabolically activates the carcinogens aflatoxin B1, a tobacco-specific nitrosamine 4-methylnitrosamino)-1-(3-pyridyl)-1-butone, and N-nitrosodiethylamine. CYP2A6 also carries out coumarin metabolism by aromatic hydroxylation in humans. Coumarin 7-hydroxylation has been used as a marker for CYP2A6 activity in vitro and the basis for measuring the in vivo expression of CYP2A6. A genetic polymorphism has been found in CYP2A6 that is due to three variant allelic forms, i.e., CYP2A6*1, 2A6*2, 2A6*3, respectively.

Cytochrome P450 2D6, also known as debrisoquine hydroxylase, is the best characterized polymorphic P450 in the human population. A poor metabolizer phenotype has been reported which behaves as an autosomat recessive trait with an incidence between 5 and 10% in the white population of North America and Europe. Poor metabolizers exhibit negligible amounts of cytochrome P450 2D6. Genetic differences in cytochrome P450 2D6 may be associated with increased risk of developing environmental and occupational based diseases.

There is some evidence that S-mephenytoin 4' hydroxylase activity resides in the cytochrome P450 2C family of enzymes. A number of 2C human variants (designated 2C8, 2C9 and 2C10) have been partially purified, and/or cloned. A comparison of the P450 2C cDNAs and their predicted amino acid sequences shows that about 70% of the amino acids are absolutely conserved among the human P450 2C subfamily. Some regions of human P450 2C protein sequences have particularly highly conservation, and these regions may participate in common P450 functions. Other regions show greater sequence divergence regions and are likely responsible for different substrate specificities between 2C members. Several drugs for treating cardiovascular and psychiatric disorders are known substrates of cytochrome P450 2D6.

### Cytochrome P450 and PSC-THP1-EP, PSC-THP1-HEP, PSC-THP1-EP-FAST, PSC-THP1-EPEP cell lines

It is predicted that the hepatocyte-like cell lines of the invention PSC-THP1-EP, PSC-THP1-HEP, PSC-THP1-EP-FAST, PSC-THP1-EPEP will be useful, like human hepatocytes, for the determination of chemicals and drugs interactions and that, as a consequence, it will be possible to measure chemicals/drug metabolism via the cytochrome P450 family.

Hepatocyte-like cell lines of the invention express cytochrome P450, as measured by DNA microarray. In particular, multiple forms of cytochrome P450 genes have been identified in the PSC-THP1-EP, PSC-THP1-HEP, PSC-THP1-EP-FAST, PSC-THP1-EPEP cell lines of the invention, as follows:
Homo sapiens cytochrome P450, family 1, subfamily A, polypeptide 2 (CYP1A2), mRNA
Homo sapiens cytochrome P450, family 1, subfamily A, polypeptide 1 (CYP1A1), mRNA
Homo sapiens cytochrome P450, family 1, subfamily B, polypeptide 1 (CYP1B1), RNA

Homo sapiens cytochrome P450, family 2, subfamily A, polypeptide 6 (CYP2A6), mRNA
Homo sapiens cytochrome P450, family 2, subfamily A, polypeptide 7 (CYP2A7), mRNA
Homo sapiens cytochrome P450, family 2, subfamily A, polypeptide 13 (CYP2A13), mRNA
Homo sapiens cytochrome P450, family 2, subfamily B, polypeptide 6 (CYP2B6), mRNA
Homo sapiens cytochrome P450, family 2, subfamily C, polypeptide 8 (CYP2C8), mRNA
Homo sapiens cytochrome P450, family 2, subfamily C, polypeptide 9 (CYP2C9), mRNA
Homo sapiens cytochrome P450, family 2, subfamily C, polypeptide 18 (CYP2C18), mRNA
Homo sapiens cytochrome P450, family 2, subfamily C, polypeptide 19 (CYP2C19), mRNA
Homo sapiens cytochrome P450, family 2, subfamily D, polypeptide 6 (CYP2D6), transcript variant 1, mRNA
Homo sapiens cytochrome P450, family 2, subfamily E, polypeptide 2 homolog (FLJ39501), mRNA
Homo sapiens cytochrome P450, family 2, subfamily E, polypeptide 1 (CYP2E1), mRNA
Homo sapiens cytochrome P450, family 2, subfamily F, polypeptide 1 (CYP2F1), mRNA
Homo sapiens cytochrome P450, family 2, subfamily J, polypeptide 2 (CYP2J2), mRNA
Homo sapiens cytochrome P450, family 2, subfamily R, polypeptide 1 (CYP2R1), mRNA
Homo sapiens cytochrome P450, family 2, subfamily S, polypeptide 1 (CYP2S1), mRNA
Homo sapiens cytochrome P450, family 2, subfamily U, polypeptide 1 (CYP2U1), mRNA
Homo sapiens cytochrome P450, family 2, subfamily W, polypeptide 1 (CYP2W1), RNA

Homo sapiens cytochrome P450, family 3, subfamily A, polypeptide 4 (CYP3A4), mRNA
Homo sapiens cytochrome P450, family 3, subfamily A, polypeptide 5 (CYP3A5), mRNA
Homo sapiens cytochrome P450, family 3, subfamily A, polypeptide 7 (CYP3A7), mRNA
Homo sapiens cytochrome P450, family 3, subfamily A, polypeptide 43 (CYP3A43), mRNA

Homo sapiens cytochrome P450, family 4, subfamily A, polypeptide 11 (CYP4A11), mRNA
Homo sapiens cytochrome P450, family 4, subfamily A, polypeptide 22 (CYP4A22), mRNA
Homo sapiens cytochrome P450, family 4, subfamily B, polypeptide 1 (CYP4B1), mRNA
Homo sapiens cytochrome P450, family 4, subfamily F, polypeptide 2 (CYP4F2), mRNA
Homo sapiens cytochrome P450, family 4, subfamily F, polypeptide 3 (CYP4F3), mRNA
Homo sapiens cytochrome P450, family 4, subfamily F, polypeptide 8 (CYP4F8), mRNA
Homo sapiens cytochrome P450, family 4, subfamily F, polypeptide 11 (CYP4F11), mRNA
Homo sapiens cytochrome P450, family 4, subfamily F, polypeptide 12 (CYP4F12), mRNA
Homo sapiens cytochrome P450, family 4, subfamily X, polypeptide 1 (CYP4X1), mRNA
Homo sapiens cytochrome P450, family 4, subfamily V, polypeptide 2 (CYP4V2), mRNA
Homo sapiens cytochrome P450, family 4, subfamily Z, polypeptide 1 (CYP4Z1), mRNA

Since the cell lines according to the invention express the above-identified forms of cytochrome P450, they are potentially useful for carrying out in vitro assays for the determination of the metabolism of drugs and chemical substances and for the determination of drug-drug interactions.

### BIBLIOGRAPHY

1. Zhao Y, Glesne D, Huberman E. (2003). A human peripheral blood monocyte-derived subset acts as pluripotent stem cells. Proc. Natl. Acad. Sci. USA. 100(5):2426-31.
2. Stem Cells: Scientific Progress and Future Research Directions. Department of Health and Human Services. June 2001.
3. Uzan G. (2004). Therapeutic use of stem cells. Rev. Prat. 54(13):1399-403.
4. Griffith, L. G. & Naughton, G. (2002). Tissue engineering-current challenges and expanding opportunities. Science. 295, 1009-1014.
5. Wagers, A. J., Christensen, J. L., & Weissman, I. L. (2002). Cell fate determination from stem cells. Gene Ther. 9, 606-612.
6. Lagasse, E., Connors, H., Al Dhalimy, M., Reitsma, M., Dohse, M., Osborne, L., Wang, X., Finegold, M., Weissman, I.L., and Grompe, M. (2000). Purified haematopoietic stem cells can differentiate into hepatocytes in vivo*.*
7. Theise, N.D., Nimmakayalu, M., Gardner, R., Illei, P.B., Morgan, G., Teperman, L., Henegariu, O., and Krause, D.S. (2000). Liver from bone marrow in humans. Hepatology. 32, 11-16.
8. Osawa, M., Hanada, K., Hamada, H., and Nakauchi, H. (1996). Long-term lymphohematopoietic reconstitution by a single CD34- low/negative haematopoietic stem cell. Science. 273, 242-245.
9. Domen, J. and Weissman, I.L. (1999). Self-renewal, differentiation or death: regulation and manipulation of haematopoietic stem cell fate. Mol. Med. Today. 5, 201-208:
10. Petersen, B.E., Bowen, W.C., Patrene, K.D., Mars, W.M., Sullivan, A.K., Murase, N., Boggs, S.S., Greenberger, J.S., and Goff, J.P. (1999). Bone marrow as a potential source of hepatic oval cells. Science. 284, 1168-1170.
11. Deutsch, G., Jung, J., Zheng, M., Lora, J., and Zaret, K.S. (2001). A bipotential precursor population for pancreas and liver within the embryonic endoderm. Development. 128, 871-881.
12. Crosby, H.A. and Strain, A.J. (2001). Adult liver stem cells: bone marrow, blood, or liver derived? Gut. 48, 153-154.
13. Bianco, P. and Cossu, G. (1999). Uno, nessuno e centomila - One, none and a hundred thousand: searching for the identity of mesodermal progenitors. Exp. Cell Res. 251, 257-263. Exp. Cell Res. 1999 Sep. 15;251(2):257-63.
14. Roy, V. and Verfaillie, C.M. (1999). Expression and function of cell adhesion molecules on foetal liver, cord blood and bone marrow haematopoietic progenitors: implications for anatomical localization and developmental stage specific regulation of haematopoiesis. Exp. Hematol. 27, 302-312.
15. Korbling, M., Katz, R. L., Khanna, A., Ruifrok, A. C., Rondon, G., Albitar, M., Champlin, R. E., & Estrov, Z. (2002). Hepatocytes and epithelial cells of donor origin in recipients of peripheral-blood stem cells. N. Engl. J. Med. 346, 738-746.
16. Tsuchiya S, Yamabe M, Yamaguchi Y, Kobayashi Y, Konno T, Tada K. Establishment and characterization of a human acute monocytic leukaemia cell line (THP-1). (1980). Int. J. Cancer; 26: 171-176.
17. Rabinovitch, M. & De Stefano, M. J. Cell shape changes induced by cationic anaesthetics. (1976). J. Exp. Med. 143, 290-304.
18. Nakabo, Y. & Pabst, M. J. Lysis of leukaemic cells by human macrophages: inhibition by 4-(2-aminoethyl)-benzenesulphonyl fluoride (AEBSF), a serine protease inhibitor. (1996). J. Leukocyte Biol. 60, 328-336.
19. Vitale, A., Guarini, A., Latagliata, R., Cignetti, A. & Foa, R. Cytotoxic effectors activated by low-dose IL-2 plus IL-12 lyse IL-2-resistant autologous acute myeloid leukaemia blasts.(1998). Br. J. Haematol. Apr;101(1):150-7.
20. Semizarov, D., Glesne, D., Laouar, A., Schiebel, K. & Huberman, E. (1998). A lineage-specific protein kinase crucial for myeloid maturation. Proc. Natl. Acad. Sci. USA 95, 15412-15417.
21. Rabinovitch, M. & De Stefano, M. J. (1976) J. Exp. Med. 143, 290-304.
22. Vessey CJ, de la Hall PM. Hepatic stem cells: a review. Pathology. 2001 May;33(2):130-41. Review. PMID: 11358043
23. Zhang Y, Bai XF, Huang CX. Hepatic stem cells: existence and origin. World J. Gastroenterol. 2003 Feb;9(2):201-4. Review. PMID: 12532431
24. Petersen BE. Hepatic Oval Cells: "Knocking on Forbidden Doors". 2000 e-biomed. Vol. 1 Mary Ann Liebert, Inc.
25. Fausto N, Campbell JS. The role of hepatocytes and oval cells in liver regeneration and repopulation. Mech. Dev. 2003 Jan;120(1):117-30. Review. PMID: 12490302
26. Overturf K, al-Dhalimy M, Ou CN, Finegold M, Grompe M. Serial transplantation reveals the stem-cell-like regenerative potential of adult mouse hepatocytes. Am. J. Pathol. 1997; 151: 1273-1280
27. Kountouras J, Boura P, Lygidakis NJ. Liver regeneration after hepatectomy. Hepatogastroenterology 2001; 48: 556-562.
28. Li, A. P., Lu, C., Brent, J. A., Pham, C., Fackett, A., Ruegg, C. E., and Silber, P. M. (1999). Cryopreserved human hepatocytes: characterization of drug-metabolizing enzyme activities and applications in higher throughput screening assays for hepatotoxicity, metabolic stability, and drug-drug interaction potential. Chem. Biol. Interact. 121, 17-35.
29. Ruegg, C. E., Silber, P. M., Mughal, R. A., Ismail, J., Lu, C., Bode, D. C., and Li, A. P. (1997). Cytochrome-P450 induction and conjugated metabolism in primary human hepatocytes after cryopreservation. In Vitro Toxicol. 10(2), 217-222.

## Claims

1. An immortal differentiated cell line obtainable from a pluripotent stem cell line derived from a human leukaemia cell line, the pluripotent stem cell line expressing on its cell surface at least the markers CD90, CD 14 and CD34, the differentiated cell line having a hepatocytic phenotype and producing albumin and blood coagulation factors, the differentiated cell line being **characterised in that** it is the cell line designated as PSC-THP1-EP deposited with the Advanced Biotechnology Centre - Interlab Cell Line Collection (ICLC), Genoa, Italy, under accession number ICLC PD No. 06005 on 10 November 2006.

2. An immortal differentiated cell line obtainable by culturing a cell line according to claim 1 in the presence of HGF (Hepatocyte Growth Factor), M-CSF (Macrophage Colony-Stimulating Growth Factor) and EGF (Epithelial Growth Factor), as well as Interleukin 2 and Interleukin 6, **characterised in that** it is the cell line designated as PSC-THPI-EP-FAST deposited with the Advanced Biotechnology Centre - Interlab Cell Line Collection (ICLC), Genoa, Italy under accession number ICLC PD No. 06008 on 15 December 2006.

3. An immortal differentiated cell line obtainable by the fusion between the human HepG2 cell line and the cell line according to claim 1, **characterised in that** it is the cell line designated as PSC-THP1-HEP deposited with the Advanced Biotechnology Centre - Interlab Cell Line Collection (ICLC), Genoa, Italy under accession number ICLC PD No. 06006 on 15 December 2006.

4. An immortal differentiated cell line obtainable by the fusion between the human HepG2 cell line, the cell line according to claim 1 and mature human primary hepatocytes, **characterised in that** it is the cell line designated as PSC-THP1-EPEP deposited with the Advanced Biotechnology Centre - Interlab Cell Line Collection, Genoa, Italy under accession number ICLC PD No. 06007 on 15 December 2006.

5. A method of preparing a cell line according to claim 2, comprising culturing the cell line according to claim 1 in the presence of HGF HGF (Hepatocyte Growth Factor), M-CSF (Macrophage Colony-Stimulating Growth Factor) and EGF (Epithelial Growth Factor), as well as Interleukin 2 and Interleukin 6.

6. A method of preparing a cell line according to claim 3, comprising the fusion between the human HepG2 cell line and the cell line according to claim 1.

7. A method of preparing a cell line according to claim 4, comprising the fusion between the human HepG2 cell line, the cell line according to claim 1 and mature human primary hepatocytes.

8. The in vitro use of a cell line according to any of claims 1 to 4 for the production of albumin.

9. The in vitro use of a cell line according to any of claims 1 to 4 for the production of one or more blood coagulation factors.

10. The use of a cell line according to any of claims 1 to 4 for carrying out an *in vitro* assay for the determination of the metabolism of drugs or for the determination of drug-drug interactions.

## Patentansprüche

1. Unsterbliche differenzierte Zelllinie, erhältlich von einer pluripotenten Stammzelllinie, die von einer humanen Leukämiezelllinie stammt, wobei die pluripotente Stammzelllinie auf ihrer Zelloberfläche mindestens die Marker CD90, CD14 und CD34 exprimiert, wobei die differenzierte Zelllinie einen Hepatozyt-Phänotyp aufweist und Albumin und Blutkoagulationsfaktoren produziert, wobei die differenzierte Zelllinie **dadurch gekennzeichnet ist, dass** es die Zelllinie ist, die bezeichnet wird als PSC-THP1-EP, hinterlegt im Advanced Biotechnology Centre - Interlab Cell Line Collection (ICLC), Genua, Italien, unter der Hinterlegungsnummer ICLC PD No. 06005, am 10. November 2006.

2. Unsterbliche differenzierte Zelllinie, erhältlich durch Kultivieren einer Zelllinie nach Anspruch 1 in der Gegenwart von HGF (Hepatozytenwachstumsfaktor (Hepatocyte Growth Factor)), M-CSF (Makrophagen-Kolonie-stimulierender Wachstumsfaktor (Macrophage Colony-Stimulating Growth Factor)) und EGF (Epithelwachstumsfaktor (Epithel Growth Factor), als auch Interleukin 2 und Interleukin 6, **dadurch gekennzeichnet**, das es die Zelllinie ist, die bezeichnet wird als PSC-THP1-EP-FAST, hinterlegt im Advanced Biotechnology Centre - Interlab Cell Line Collection (ICLC), Genua, Italien, unter der Hinterlegungsnummer ICLC PD No. 06008, am 15. Dezember 2006.

3. Unsterbliche differenzierte Zelllinie, erhältlich durch die Fusion zwischen der humanen HepG2-Zelllinie und der Zelllinie gemäß Anspruch 1, **dadurch gekennzeichnet, dass** es die Zelllinie ist, die bezeichnet wird als PSC-THP1-HEP, hinterlegt im Advanced Biotechnology Centre - Interlab Cell Line Collection (ICLC), Genua, Italien, unter der Hinterlegungsnummer ICLC PD No. 06006, am 15. Dezember 2006.

4. Unsterbliche differenzierte Zelllinie, erhältlich durch die Fusion zwischen der humanen HepG2-Zelllinie, der Zelllinie gemäß Anspruch 1 und reifen humanen primären Hepatozyten, **dadurch gekennzeichnet, dass** es die Zelllinie ist, die bezeichnet wird als PSC-THP1-EPEP, hinterlegt im Advanced Biotechnology Centre - Interlab Cell Line Collection (ICLC), Genua, Italien, unter der Hinterlegungsnummer ICLC PD No. 06007 am 15. Dezember 2006.

5. Verfahren zum Herstellen einer Zelllinie nach Anspruch 2, umfassend das Kultivieren der Zelllinie nach Anspruch 1 in der Gegenwart von HGF (Hepatozytenwachstumsfaktor (Hepatocyte Growth Factor)) M-CSF (Makrophagen-Kolonie-stimulierender Wachstumsfaktor (Macrophage Colony-Stimulating Growth Factor)) und EGF (Epithelwachstumsfaktor (Epithel Growth Factor), als auch Interleukin 2 und Interleukin 6.

6. Verfahren zum Herstellen einer Zelllinie nach Anspruch 3, umfassend die Fusion zwischen der humanen HepG2-Zelllinie und der Zelllinie nach Anspruch 1.

7. Verfahren zum Herstellen einer Zelllinie nach 4, umfassend die Fusion zwischen der humanen HepG2-Zelllinie, der Zelllinie nach Anspruch 1 und reifen humanen primären Hepatozyten.

8. In vitro-Verwendung einer Zelllinie nach einem der Ansprüche 1 bis 4 zur Produktion von Albumin.

9. In vitro-Verwendung einer Zelllinie nach einem der Ansprüche 1 bis 4 zur Produktion von einem oder mehreren Blutkoagulationsfaktoren.

10. Verwendung einer Zelllinie nach einem der Ansprüche 1 bis 4 zum Durchführen eines in vitro-Assays zur Bestimmung des Metabolismus von Arzneimitteln oder zur Bestimmung von Arzneimittel-Arzneimittelwechselwirkungen.

## Revendications

1. Lignée cellulaire immortelle différenciée susceptible d'être obtenue à partir d'une lignée de cellules souches pluripotentes dérivée d'une lignée de cellules de leucémie humaine, la lignée de cellules souches pluripotentes exprimant sur sa surface cellulaire au moins les marqueurs CD90, CD14 et CD34, la lignée cellulaire différenciée ayant un phénotype hépatocytaire et produisant de l'albumine et des facteurs de coagulation sanguine, la lignée cellulaire différenciée étant **caractérisée en ce qu'**il s'agit de la lignée cellulaire désignée PSC-THP1-EP déposée auprès du *Advanced Biotechnology Centre - Interlab Cell Line Collection (ICLC),* Gênes, Italie, sous le numéro d'ordre ICLC PD n° 06005 le 10 novembre 2006.

2. Lignée cellulaire immortelle différenciée susceptible d'être obtenue en mettant en culture une lignée cellulaire selon la revendication 1 en présence de HGF (facteur de croissance des hépatocytes), de M-CSF (facteur de croissance stimulant les colonies de macrophages) et d'EGF (facteur de croissance épithéliale), ainsi que d'Interleukine 2 et d'Interleukine 6, **caractérisée en ce qu'**il s'agit de la lignée cellulaire désignée PSC-THP1-EP-FAST déposée auprès du *Advanced Biotechnology Centre - Interlab Cell Line Collection* (*ICLC*), Gênes, Italie, sous le numéro d'ordre ICLC PD n° 06008 le 15 décembre 2006.

3. Lignée cellulaire immortelle différenciée susceptible d'être obtenue par la fusion entre la lignée cellulaire HepG2 humaine et la lignée cellulaire selon la revendication 1, **caractérisée en ce qu'**il s'agit de la lignée cellulaire désignée PSC-THP1-HEP déposée auprès du *Advanced Biotechnology Centre - Interlab Cell Line Collection* (*ICLC*), Gênes, Italie, sous le numéro d'ordre ICLC PD n° 06006 le 15 décembre 2006.

4. Lignée cellulaire immortelle différenciée susceptible d'être obtenue par la fusion entre la lignée cellulaire HepG2 humaine, la lignée cellulaire selon la revendication 1 et des hépatocytes primaires humains matures, **caractérisée en ce qu'**il s'agit de la lignée cellulaire désignée PSC-THP1-EPEP déposée auprès du *Advanced Biotechnology Centre - Interlab Cell Line Collection* (*ICLC*), Gênes, Italie, sous le numéro d'ordre ICLC PD n° 06007 le 15 décembre 2006.

5. Procédé de préparation d'une lignée cellulaire selon la revendication 2, comprenant la mise en culture de la lignée cellulaire selon la revendication 1 en présence de HGF (facteur de croissance des hépatocytes), de M-CSF (facteur de croissance stimulant les colonies de macrophages) et d'EGF (facteur de croissance épithéliale), ainsi que d'Interleukine 2 et d'Interleukine 6.

6. Procédé de préparation d'une lignée cellulaire selon la revendication 3, comprenant la fusion entre la lignée cellulaire HepG2 humaine et la lignée cellulaire selon la revendication 1.

7. Procédé de préparation d'une lignée cellulaire selon la revendication 4, comprenant la fusion entre la lignée cellulaire HepG2 humaine, la lignée cellulaire selon la revendication 1 et des hépatocytes primaires humains matures.

8. Utilisation *in vitro* d'une lignée cellulaire selon l'une quelconque des revendications 1 à 4 pour la production d'albumine.

9. Utilisation *in vitro* d'une lignée cellulaire selon l'une quelconque des revendications 1 à 4 pour la production d'un ou plusieurs facteurs de coagulation sanguine.

10. Utilisation d'une lignée cellulaire selon l'une quelconque des revendications 1 à 4, pour la mise en oeuvre d'un essai *in vitro* pour la détermination du métabolisme de substances médicamenteuses ou pour la détermination d'interactions substance médicamenteuse-substance médicamenteuse.
